(19) Europäisches Patentamt
European Patent Office
Office européen des brevets

(11) Publication number: 0 233 403 B1

(12) # EUROPEAN PATENT SPECIFICATION

(45) Date of publication of patent specification: 02.01.91

(51) Int. Cl.[5]: C 07 C 245/12, C 07 C 249/16, C 07 H 19/20, C 07 B 63/02, B 01 J 19/12

(21) Application number: 86309271.4

(22) Date of filing: 27.11.86

(54) **Photo-labile protecting agents and method.**

(30) Priority: 02.12.85 GB 8529684

(43) Date of publication of application:
26.08.87 Bulletin 87/35

(45) Publication of the grant of the patent:
02.01.91 Bulletin 91/01

(84) Designated Contracting States:
DE FR GB IT

(56) References cited:
EP-A-0 187 039
DE-A-2 335 107

Patent Abstracts of Japan, unexamined applications, C section, vol. 3, no. 147, Dec 5, 1979; The Patent Office Japanese Government page 98 c 66

Biochemistry, vol. 17, no. 10/78; A Publication of the American Chemical Society, Jack H. Kaplan et al." Rapid Photolytic Release of Adenosine 5'- Triphosphate from a Protected Analogue: Utilization by the Na:K Pump of Human Red Blood Cell Ghosts" pages 1929-1935

(73) Proprietor: MEDICAL RESEARCH COUNCIL
20 Park Crescent
London W1N 4AL (GB)

(72) Inventor: Trentham, David Rostron
c/o Medical Research Council 20 Park Crescent
London W1N 4AL (GB)
Inventor: Reid, Gordon Paterson
c/o Medical Research Council 20 Park Crescent
London W1N 4AL (GB)

(74) Representative: Pennant, Pyers et al
Stevens, Hewlett & Perkins 5 Quality Court
Chancery Lane
London, WC2A 1HZ (GB)

EP 0 233 403 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to certain diazo compounds, many of which are new compounds per se, and to their use as photo-labile protecting agents for organic compounds. The invention will provide a useful new way for preparing protected or 'caged' organic compounds which can be introduced into biological systems and there released by means of light radiation. But the invention is likely to be applicable also in other areas, for example in organic synthesis and purification.

2-nitrobenzyl derivatives have been used for many years as photo-labile protecting groups in synthetic organic chemistry. Indeed, 2-nitrobenzaldehyde tosylhydrazone, a precursor of 2-nitrophenyl-diazomethane, is commercially available for this purpose. Such compounds are not suitable for use in biological systems, because photolysis gives rise to a reactive benzaldehyde compound and in certain cases the compounds themselves are susceptible to attack by endogenous thiols.

A caged derivative of adenosine 5'-triphosphate (ATP) has been described, in which the caging group is 1-(2-nitro)phenylethyl, and has been shown to act as photo-labile source of ATP (Kaplan et al (1978) Biochemistry 17, pages 1929 to 1935). Preparation of caged ATP involves reaction of ADP morpholidate with 1-(2-nitro)phenylethyl phosphate by the following reaction.

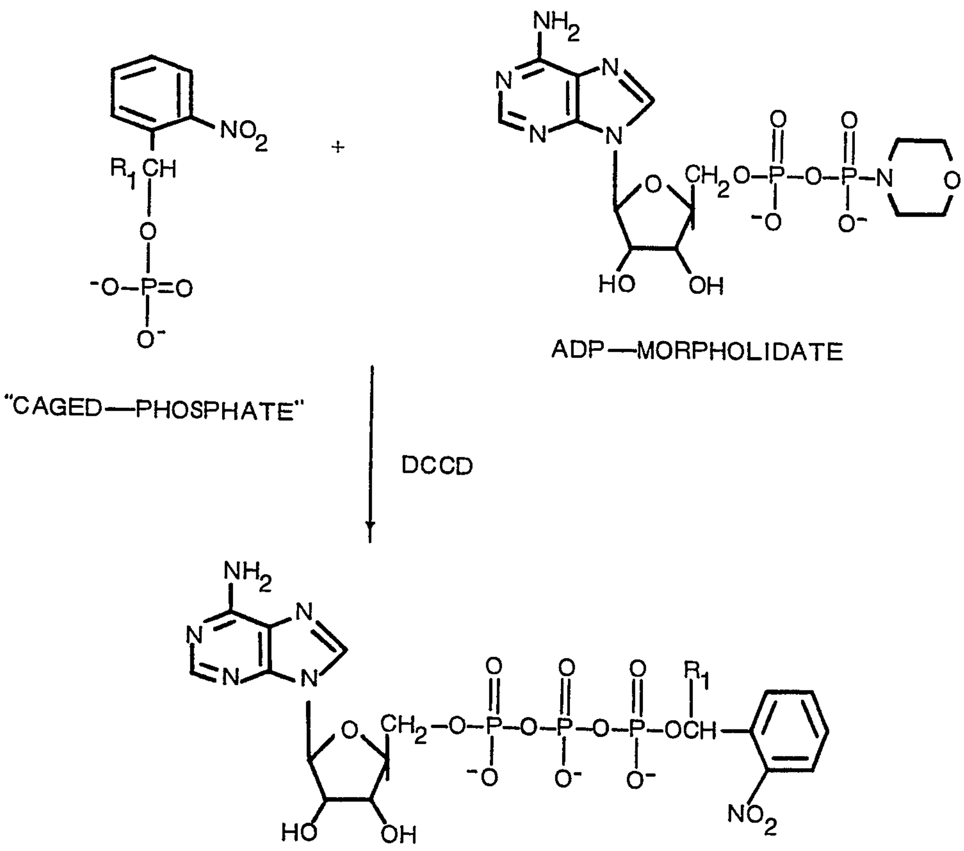


"CAGED—PHOSPHATE"

ADP—MORPHOLIDATE

"CAGED–ATP"

The method is labour-intensive and gives rise to low yields. In particular, it is difficult to make radioactively labelled caged ATP, such as is constantly required for biological experiments, although this has been achieved (Ferenczi et al, J. Physiol. (1984), 352, pages 575 to 599). There has long been a need for a better method of making caged ATP, and in particular for a method that can readily be used to make caged radiolabelled ATP.

In one aspect this invention provides a diazo compound having the formula

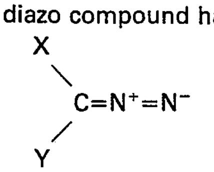

where X is an optionally substituted aromatic group which carries a —$NO_2$ group in the ortho-position, and Y is an optionally substituted hydrocarbon group. As described below, these compounds can be reacted with ATP, or with another organic compound having a reactive hydrogen atom, to provide a caged compound.

The group X may be 2-nitrophenyl. The phenyl group may carry hydrogen or other electron-donating substituents to assist rapid photolysis. Or the phenyl group may carry one or more groups such as alkoxy groups to increase absorption of light during photolysis or to shift the optimum wavelength for photolysis to longer wavelengths. Examples of diazo compounds falling within the above definition are:—

1-(2-nitro)phenyl-diazoethane (I)

I

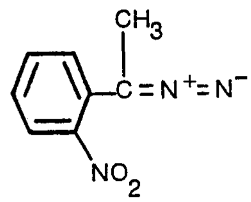

1-(2-nitro-4,5-dimethoxy)phenyl-diazoethane (II)

II

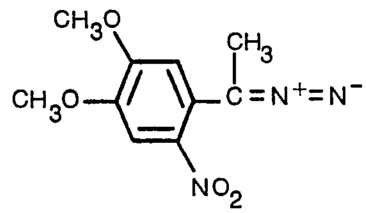

1-(2-nitro-3,4,5,6-tetramethyl)phenyl-diazoethane (III)

III

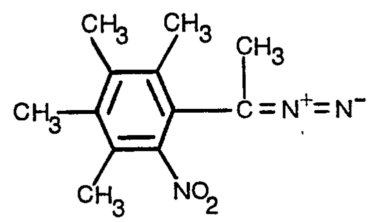

To the best of our knowledge these three, together with others falling within the above definition, are new compounds. Generally they lead to caged compounds with an asymmetric carbon atom. If the parent compound is a chiral molecule (such as ATP, see above), this will lead to a caged compound (e.g. caged ATP) that exists as two diastereoisomers. (In the case of caged ATP these two diastereoisomers have been separated and shown to have identical photochemical properties, so that, in general, separation of diastereoisomers is not necessary).

The diazo compounds may be prepared from the corresponding ketones by the following two-step method:—

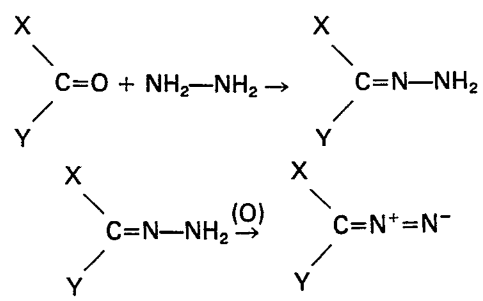

In the first step, the ketone is reacted with hydrazine hydrate to form the corresponding hydrazone. These hydrazone intermediates are believed to be new compounds, and form a further aspect of this invention. Since they are more stable than the corresponding diazo compounds, to which they may readily be converted by oxidation, they provide a convenient form in which the diazo compounds may be stored, transported and sold. In the second step, the hydrazone may be oxidized to the corresponding diazo compound using known chemistry.

According to another aspect, the invention provides a method which comprises reacting an organic compound having a reactive hydrogen atom (or an organic compound in its conjugate base form) with a diazo compound as defined above. The reaction may be shown as follows, where RH is the organic compound concerned:

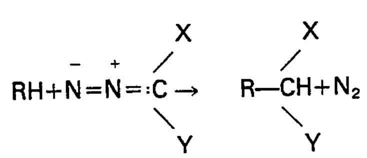

The reactive hydrogen atom may for example be part of a group selected from phosphate, thiophosphate, phosphonate, carboxyl and phenol. The organic compound may be used in its conjugate base form, that is to say with the reactive hydrogen atom ionized, though the protonated form is generally believed to be the reactive species. The reaction proceeds easily under a wide range of conditions. For hydrophilic compounds of biological interest, the reaction is preferably performed with shaking in a water/organic solvent mixture, using for example chloroform or ether as the water-immiscible organic solvent. For example, reaction with ATP and other nucleotides in a shaken water/chloroform mixture at ambient temperature overnight is found to give the caged compounds in high yield and with excellent selectivity. Reaction temperature is not critical. For organic compounds in which the reactant or product is not soluble or dispersible in water, it may be preferable to use a single-phase organic reaction medium. Examples 13 and 14 illustrate this. On occasion (for example in the case of cyclic nucleotides listed below) a single-phase organic reaction is desirable because water competes with the reactant for the diazo compound. The caged product is readily recovered from the reaction mixture and purified, for example by means of HPLC. On occasion more than one group is caged. If it is desired that only one group should be caged, standard chemical procedures can be used to obtain the desired product. Examples 12 and 13 illustrate this. Caged compounds prepared by these methods may, if necessary, be further modified. Example 15 illustrates this. The following is a list of biologically significant compounds that can be caged by the method of this invention.

1. Phosphates, thiophosphates and phosphonates
ribonucleotides
e.g. adenosine 5′ triphosphate
guanosine 5′ diphosphate
cytosine 5′monophosphate

deoxyribonucleotides
e.g. deoxyadenosine 5′triphosphate
deoxythymidine 5′ diphosphate
deoxyguanosine monophosphate

nucleotide analogues
e.g. 2′,3′-dideoxythymidine 5′-triphosphate
cytosine-β-D-arabinofuranoside 5′-triphosphate
1,$N^6$-ethenoadenosine 5′-triphosphate
1,$N^6$-etheno-2-aza-adenosine 5′-triphosphate
adenylyl-imidodiphosphate (ATP (β,γ-NH))
adenosine 5′-0-(3-thiotriphosphate) (ATP(γS))
adenylyl-(β,γ-methylene)diphosphonate (ATP(β,γ-$CH_2$))
GTP(β,γ-NH)
GTP(γS)
GTP(β,γ-$CH_2$)

oligonucleotides

cyclic nucleotides
e.g. cyclic adenosine 3′,5′ monophosphate (cAMP),
cGMP,
cCMP

cyclic nucleotide analogues
e.g. cyclic adenosine 3′,5′-monophosphothioate,
cyclic guanosine 3′,5′-monophosphothioate
cyclic formycin 3′,5′-monophosphate

Sugar phosphates
e.g. glucose 1-phosphate
glucose 6-phosphate

inositol triphosphate
and related compounds
e.g. phosphatidylinositolbis-phosphate ($PIP_2$),
phosphatidylinositol monophosphate ($PIP_1$),
phosphatidylinositol,
inositol tetraphosphate ($IP_4$),
inositol bisphosphate ($IP_2$),

inositol phosphate ($IP_1$),
inositol 1,4,5-trisphosphate,
inositol 1,3,4-trisphosphate

Phosphates in proteins and peptides

Phosphonate receptor agonists and antagonists e.g. 2-amino-4-phosphono-butyric acid, 2-amino-4-phosphono-valeric acid

vitamin derivatives
e.g. pyridoxal phosphate

Phosphoadenosylphosphosulphate (PAPS)

2. Carboxyl and phenolic groups,

amino acids
e.g. glycine

peptides
e.g. enkephalins

neurotransmitters
e.g. γ-aminobutyric acid

catecholamines

fatty acids

thromboxanes

prostaglandins

fluorescein

Of course, where a radioactively labelled caged compound is required, a radioactively labelled starting compound with a reactive hydrogen atom would be used. The synthetic method described here is far easier and has much higher yield for radiochemicals than the earlier method used for caged ATP (Ferenczi et al., J. Physiol. *352,* 575—599, 1984).

The resulting caged compounds are in many instances known, as are the conditions required for their photolysis. Thus for example, caged ATP and its photolysis are described in the two references quoted above. Light from 300 to 350 nm is suitable for this photolysis, such as may be generated by a xenon-arc flash lamp or a 347 nm frequency doubled ruby laser. A 10 millisecond exposure from a filtered (300 to 350 nm) mercury or xenon-arc lamp source is perfectly adequate when high time resolution is not required. The quantum yield for photolysis of caged ATP is about 0.7, and for any caged phosphate diester is in the range 0.3 to 0.7. When diazo compounds having alkoxy, e.g. methoxy, groups have been used, an energy input of from 1 to 5 mJ is typically sufficient for photolysis; when the diazo compound did not contain methoxy groups, an energy input of from 10 to 50 mJ may typically be required.

Applications of the method of this invention all involve the following steps:—

a) Providing a caged organic compound which is the reaction product of an organic compound having a reactive hydrogen atom with a diazo compound as defined.

b) Doing something to the caged organic compound, for example changing its chemical identity or its environment.

c) Illuminating the caged compound to effect photolysis.

Several applications of this general technique may be noted:—

i) The caged compound is introduced to a biological system, and is there photochemically decomposed so as to form the organic compound in situ in the biological system. Techniques of this kind are well known and will not be described here. The contribution of this invention is to provide a better method, and in many cases perhaps the only feasible method, of making caged compounds.

ii) The technique may be used to purify a desired compound. For example, a biological mixture may be reacted with one of the diazo compounds, so as to form a caged compound which may then be separated, e.g. by HPLC, from the remainder of the mixture. After recovery from the column and desalting the solution, the pure compound may simply be recovered by photolysis.

iii) The technique may be used in organic synthesis. For example, a reactive group of a starting organic compound may be caged or protected by reaction with a diazo compound as defined. Then the organic

compound may be modified by reaction of some other part of the molecule so as to form a protected product compound, which is subsequently recovered by photolysis. This technique is well known in the art and other photolysable protecting groups have been proposed for the purpose. The contribution of this invention is to provide a new photolysable protecting compound and method which may have advantages in certain instances.

Reference is directed to the accompanying drawings, in which:—

Figure 1 is an infrared spectrum of the product of Example 1, o-nitroacetophenone hydrazone.

Figure 2 is an I.R. spectrum of the product of Example 2, 1-(2-nitrophenyl)-diazoethane.

Figure 3 is an HPLC analysis using C-18 (solvent 10 mM inorganic phosphate at pH 5.5, 85%: methanol 15%) of the product of Example 3; absorbent detection at 254 mm; and

Figure 4 is HPLC analysis of the same product after photolysis.

The following examples illustrate the invention. In all cases the amounts of reactants used can be readily scaled up or down.

## Example 1

Synthesis of o-nitroacetophenone hydrazone

o-nitroacetophenone hydrazone was prepared by an acid catalysed reaction of o-nitroacetophenone with hydrazine hydrate.

(0.112 moles) 5.62 g hydrazine hydrate and (0.056 moles) 3.2 ml glacial acetic acid were added to (0.05 moles) 8.26 g O-nitroacetophenone in 100 ml ethanol and heated under reflux for 2.5 hour. The ethanol was evaporated off under vacuum and the product examined by I.R. (Figure 1). Disappearance of a 1690 $cm^{-1}$ band in the I.R. due to C=0 stretch was taken as evidence of complete reaction.

The solution was evaporated to dryness and dissolved in diethyl ether. A precipitate of, probably, hydrazine acetate was filtered off and the ether solution containing the hydrazone used in next reaction.

## Example 2

Synthesis of 1-(2-nitrophenyl)diazoethane

The o-nitroacetophenone hydrazone was oxidised with activated manganese dioxide by the method of Jugelt and Berseck. The ether solution was stirred with 11.3 g manganese dioxide for 3 hours at room temperature in the dark. The manganese dioxide was filtered off and the ether solution partitioned with water adjusted to pH 7 with sodium bicarbonate. The separated ether phase was dried with magnesium sulfate and stored in a tightly stoppered flask covered with tin foil to shield from the light.

The wine coloured ether solution was examined after evaporating the ether by I. R. (Figure 2). A peak at 2050$cm^{-1}$ was observed and attributed to the diazo group. The product dissolved in $CHCl_3$ was used directly in the subsequent esterifications.

Jugelt W. and Berseck TETRAHEDRON *26*, 5581 (1970)

## Example 3

Synthesis of 1-(2-nitrophenyl)ethyl esters

ATP was treated with Dowex $H^+$ to yield the $H^+$ form which was adjusted to pH 4—5 with NaOH. 3 ml of an aqueous solution containing from 1—100 u mole of the phosphate ester was stirred vigorously with 3 ml of a $CHCl_3$ solution containing 0.4 m mole 1-(2-nitrophenyl)diazoethane at room temperature for 15 hour. The product was analyzed by HPLC (generally C-18 reverse phase column) and shown to be esterified in 75—100% yield. After the $CHCl_3$ phase had been separated off, the phosphate diester was purified by C-18 reverse phase chromatography typically in 10 mM inorganic phosphate pH 5.5, Methanol (85:15 v/v). The solution containing the pure compound is adjusted to pH 7.5 and is directly loaded onto a DEAE column and eluted using a gradient of triethylamine bicarbonate in water. The solution containing the pure compound is rotary evaporated to remove excess triethylamine/bicarbonate leaving the pure compound. Care is taken to shield the compound from daylight, though in practice insignificant photolysis occurs in normal daylight. Figure 3 shows the result of HPLC analysis of the initial product before purification. Figure 4 shows HPLC analysis of the mixture produced on photolysis of the product. The caged ATP appears as a double peak (due to stereo isomerism) at about 6 minutes; the ATP formed on photolysis appears as a peak around 2 minutes.

Other caged compounds were prepared and characterized by the general method described in Example 3.

## Examples

4 P-1-(2-nitro)phenylethylguanosine 5′-phosphate (caged 5′-GMP)

5 $P^3$-1-(2-nitro)phenylethyl-2′,3′-dideoxythymidine 5′-triphosphate (caged dideoxy TTP)

6 $P^3$-1-(2-nitro)phenylethylcytidine 5′-triphosphate (caged CTP)

7 $P^3$-1-(2-nitro)phenylethyladenosine 5′-0-(3-thiotriphosphate) (caged ATP(γS)) (In caged ATP(γS) the caged group is attached to the ATP(γS) either through sulphur or non-bridging oxygen atom bonded to γ-phosphorus atom of ATP. The 'S-caged ATP(γS)' is separable from the 'O-caged ATP(γS)' by DEAE-anion exchange chromatography. The compounds have similar photochemical properties, each forming ATP(γS) on photolysis.)

8 $P^3$-1-(2-nitro)phenylethylguanosine 5′-O-(3-thiotriphosphate) (caged GTP(δS))
9 $P^3$-1-(2-nitro)phenylethyladenylylimidodiphosphate (caged ATP (β,γ-NH))
10 $P^3$-1-(2-nitro)phenylethylguanylylimidodiphosphate (caged GTP (β,γ-NH))
11 $P^3$-1-(2-nitro)phenylethyl-[2-$^3$H]adenosine 5′-triphosphate ([2-$^3$H]caged ATP)

Example 12

Inositol, 1,4,5-triphosphate was reacted with 1-(2-nitrophenyl)diazoethane by the method of Example 3. There was obtained a mixture of caged inositol triphosphates, including the 1- and 4- and 5- single caged compounds, the 1,4- and the 1,5- and the 4,5-doubly caged compounds, and the 1,4,5-triply caged compound. Reaction for only 4 hours favoured formation of the mixed singly caged compounds. Reaction for 15 hours favoured formation of the triply caged compounds.

In the reaction to form the single caged compound the 1-compound forms about four times more rapidly than the 4- or 5-compound. On the other hand if the 1,4,5-triply caged compound is partially photolyzed the 1-, 4- and 5-compounds are each formed in equal amounts. Thus to form the 1-compound the straightforward procedure is used, while to form the 4- and 5- compounds it is preferably to photolyze partially the 1,4,5-triply caged compound. The single caged isomers may be separated by anion exchange chromatography.

Example 13

Fluorescein was reacted in dimethylsulphoxide with 1-(2-nitro-4,5-dimethoxyphenyl)diazoethane as in Example 3 except for the change of solvent. The product was caged on both its carboxy and phenoxy group. The caged group was removed from the carboxy group by leaving the material in 0.1N sodium hydroxide in dimethyl sulphoxide water at 55°C for 30 minutes, leaving fluorescein caged on just the phenoxy group. The compound was purified by preparative thin layer chromatography.

Example 14

P-1-(2-nitro)phenylethyl cyclic adenosine

3′,5′-monophosphate: Cyclic adenosine 3′,5′-monophosphate was reacted in dimethyl sulphoxide with 1-(2-nitrophenyl)diazoethane as in Example 3 except for the change of solvent. The caged product was purified by preparative thin layer chromatography.

Example 15

$P^3$-1-(2-nitro)phenylethyl-1-$N^6$-ethenoadenosine 5′-triphosphate: Caged ATP (Example 3) was treated with chloroacetaldehyde as described by Secrist, Barrio, Leonard and Weber (Biochemistry *11*, 3499—3506, 1972) to form the desired product which was then isolated according to procedures outlined in Example 3.

**Claims**

1. A diazo compound having the formula

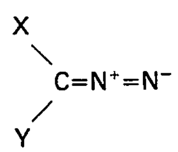

where X is an optionally substituted aromatic group which carries a $NO_2$ group in the ortho-position and Y is an optionally substituted hydrocarbon group.

2. A hydrazone compound having the formula

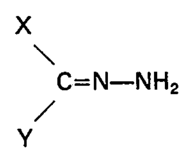

where X and Y are as defined in claim 1.

3. A compound as claimed in claim 1 or claim 2, wherein X is o-nitrophenyl.

4. A compound as claimed in any one of claims 1 to 3, wherein Y is methyl.

5. A method which comprises reacting an organic compound having a reactive hydrogen atom, or an organic compound in the conjugate base form, with a diazo compound as claimed in any of claims 1, 3 or 4.

6. A method as claimed in claim 5, wherein the reactive hydrogen atom is part of a group selected from phosphate, thiophosphate, phosphonate, carboxyl, and phenol.

7. A method as claimed in claim 5 or claim 6, wherein the reaction is performed in a shaken mixed water/organic solvent system.

8. A method which comprises photochemically decomposing the reaction product of any one of claims 5 to 7.

9. A method comprising the steps:—

providing a caged organic compound which is the reaction product of an organic compound having a reactive hydrogen atom with a diazo compound as claimed in any one of claims 1, 3 or 4.

introducing the caged organic compound into a biological system and,

photochemically decomposing the caged organic compound so as to form the organic compound in situ in the biological system.

10. A purification method comprising the steps:—

providing a starting mixture containing an organic compound having a reactive hydrogen atom and reacting the said organic compound with a diazo compound as claimed in any one of claims 1, 3 or 4, so as to form a protected compound in the mixture,

subjecting the mixture to chromatography and recovering the protected organic compound in purified form, and

photochemically decomposing the protected compound and recovering the purified organic compound.

**Patentansprüche**

1. Diazo-Verbindung der Formel

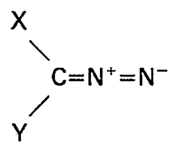

worin X eine gegebenenfalls substituierte aromatische Gruppe ist, die eine $NO_2$-Gruppe in der Ortho-Position trägt und Y eine gegebenenfalls substituierte Kohlenwasserstoff-Gruppe ist.

2. Hydrazon-Verbindung der Formel

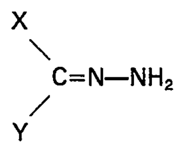

worin X und Y wie in Anspruch 1 definiert sind.

3. Verbindung nach Anspruch 1 oder Anspruch 2, worin X o-Nitrophenyl ist.

4. Verbindung nach einem der Ansprüche 1 bis 3, worin Y Methyl ist.

5. Verfahren, das das Umsetzen einer organischen Verbindung, die ein reaktives Wasserstoffatom aufweist, oder einer organischen Verbindung in der konjugierten Basenform mit einer Diazo-Verbindung nach einem der Ansprüche 1, 3 oder 4 umfaßt.

6. Verfahren nach Anspruch 5, worin das reaktive Wasserstoffatom Teil einer Gruppe ausgewählt unter Phosphat, Thiophosphat, Phosphonat, Carboxyl und Phenol ist.

7. Verfahren nach Anspruch 5 oder Anspruch 6, worin die Reaktion in einem geschüttelten gemischten Wasser/organisches Lösungsmittel-System durchgeführt wird.

8. Verfahren, das photochemisches Zersetzen des Reaktionsprodukts nach einem der Ansprüche 5 bis 7 umfaßt.

9. Verfahren, das die folgenden Schritte umfaßt: Zur-Verfügung-Stellen einer käfigartig umschlossenen organischen Verbindung, die das Reaktionsprodukt einer ein reaktives Wasserstoffatom aufweisenden organischen Verbindung mit einer Diazo-Verbindung nach einem der Ansprüche 1, 3 oder 4 ist, Einführen der käfigartig umschlossenen organischen Verbindung in ein biologisches System und, photochemisches Zersetzen der käfigartig umschlossenen organischen Verbindung, um die organische Verbindung in situ in dem biologischen System zu bilden.

10. Reinigungsverfahren, das die folgenden Schritte umfaßt: Liefern einer Ausgangsmischung, die eine organische Verbindung mit einem reaktiven Wasserstoffatom enthält und Umsetzen der organischen Verbindung mit einer Diazo-Verbindung nach einem der Ansprüche 1, 3 oder 4, um eine geschützte Verbindung in der Mischung zu bilden, Durchführen einer Chromatographie an der Mischung und Rückgewinnen der geschützten organischen Verbindung in gereinigter Form und Photochemisches Zersetzen der geschützten Verbindung und Rückgewinnen der gereinigten organischen Verbindung.

**Revendications**

1. Composé diazoïque de formule

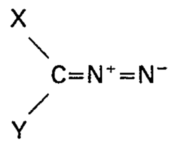

dans laquelle X est un groupe aromatique éventuellement substitué qui porte un groupe $NO_2$ en position ortho et Y est un groupe hydrocarboné éventuellement substitué.

2. Composé hydrazone de formule

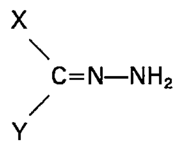

dans laquelle X et Y sont tels que définis dans la revendication 1.

3. Composé selon la revendication 1 ou 2, dans lequel X est un groupe o-nitrophényle.

4. Composé selon l'une quelconque des revendications 1 à 3, dans lequel Y est un groupe méthyle.

5. Procédé qui comprend la réaction d'un composé organique comportant un atome d'hydrogène réactif, ou d'un composé organique sous forme de base conjuguée, avec un composé diazoïque selon l'une quelconque des revendications 1, 3 ou 4.

6. Procédé selon la revendication 5, dans lequel l'atome d'hydrogène réactif fait partie d'un groupe choisi parmi un phosphate, un thiophosphate, un phosphonate, un carboxyle et un phénol.

7. Procédé selon la revendication 5 ou 6, dans lequel la réaction s'effectue dans un système mixte eau/ solvant organique agité.

8. Procédé qui comprend la décomposition photochimique du produit de réaction de l'une quelconque des revendications 5 à 7.

9. Procédé comprenant les étapes qui consistent à:

obtenir un composé organique bloqué qui est le produit de réaction d'un composé organique comportant un atome d'hydrogène réactif avec un composé diazoïque selon l'une quelconque des revendications 1, 3 ou 4,

introduire le composé organique bloqué dans un système biologique, et

décomposer par voie photochimique le composé organique bloqué de manière à former le composé organique in situ dans un système biologique.

10. Procédé de purification comprenant les étapes qui consistent à:

obtenir un mélange de départ contenant un composé organique comportant un atome d'hydrogène réactif et faire réagir ledit composé organique avec un composé diazoïque selon l'une quelconque des revendications 1, 3 ou 4, de manière à former un composé protégé dans le mélange,

soumettre le mélange à une chromatographie et récupérer le composé organique protégé sous forme purifiée, et

décomposer par voie photochimique le composé protégé et récupérer le composé organique purifié.

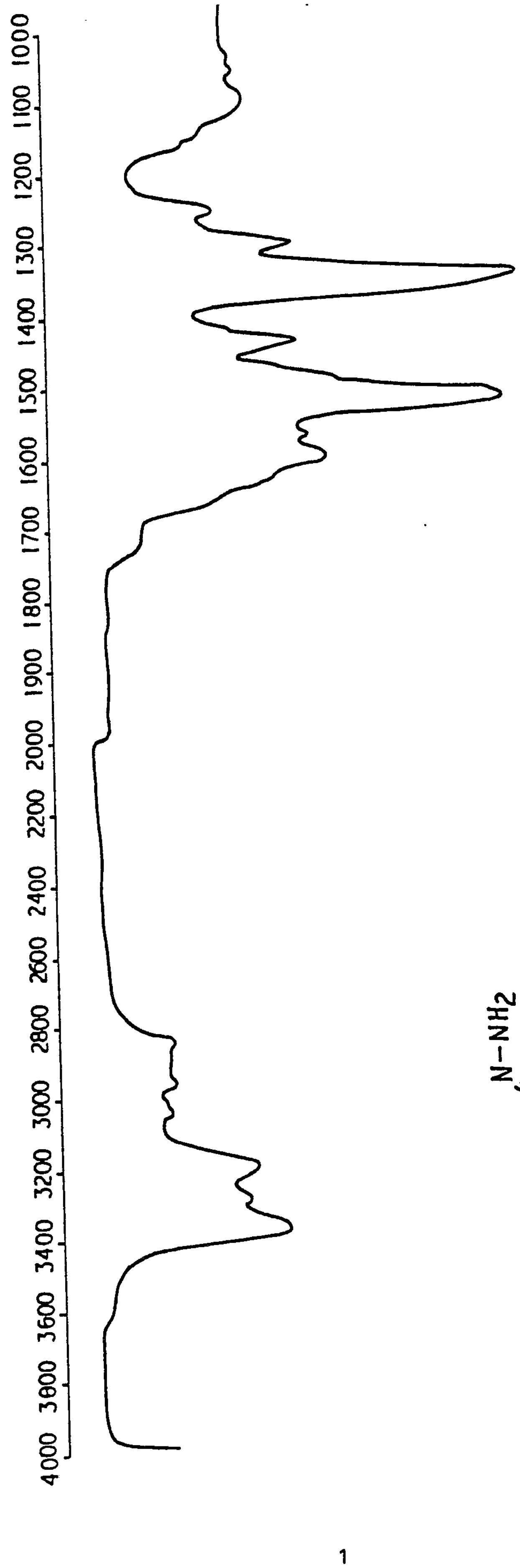
1000
1100
1200
1300
1400
1500
1600
1700
1800
1900
2000
2200
2400
2600
2800
3000
3200
3400
3600
3800
4000

FIG. 1

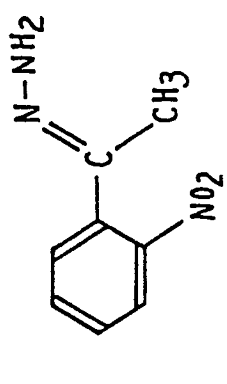

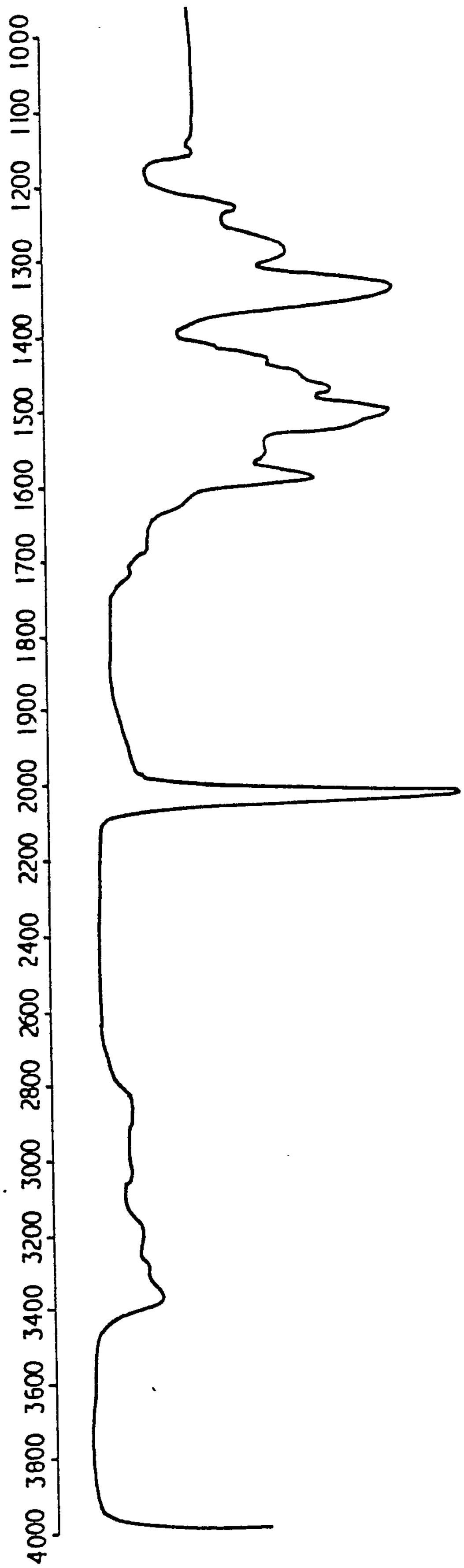
1000
1100
1200
1300
1400
1500
1600
1700
1800
1900
2000
2200
2400
2600
2800
3000
3200
3400
3600
3800
4000

FIG. 2

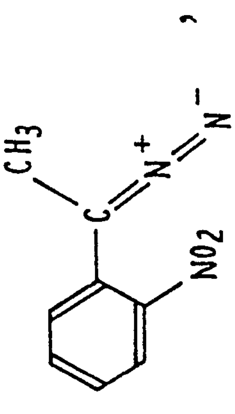

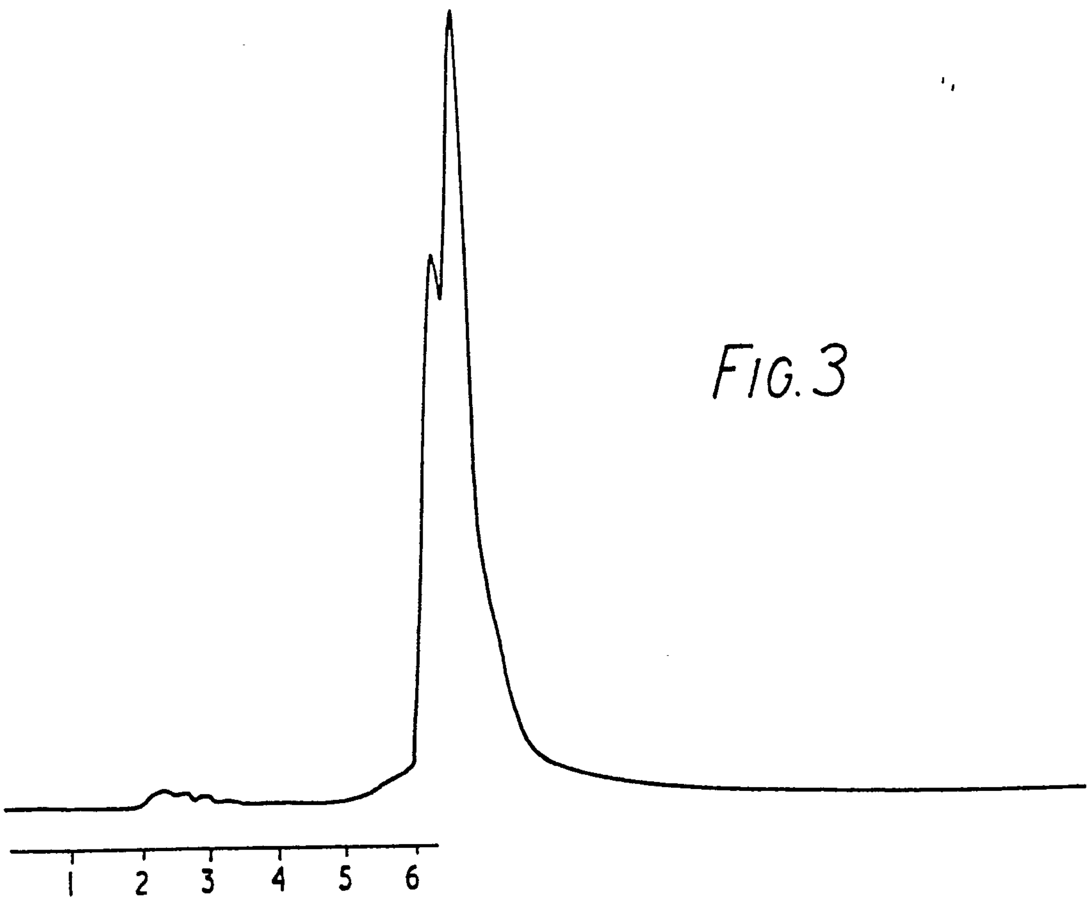


FIG. 3

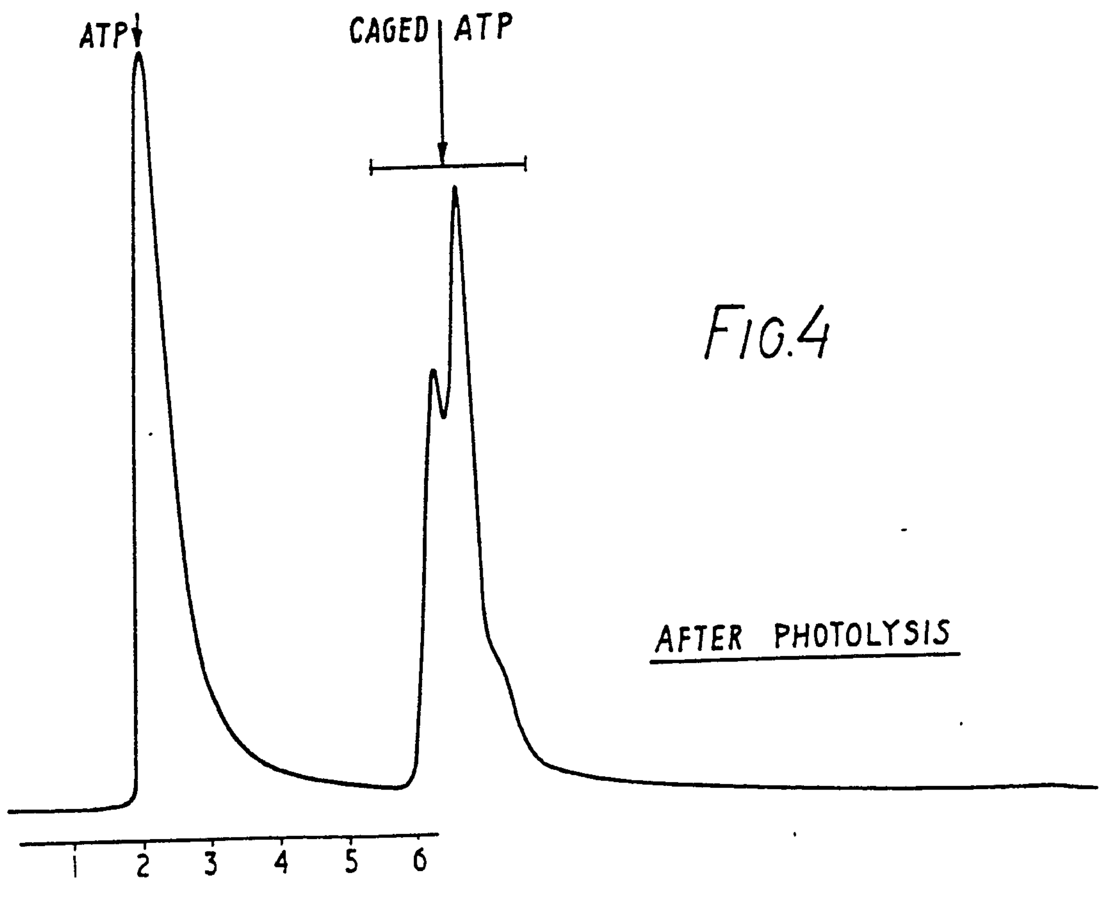


FIG. 4

AFTER PHOTOLYSIS